# EUROPEAN PATENT APPLICATION

(11) **EP 2 604 589 A1**
(43) Date of publication of application: **19.06.2013**
(21) Application number: 12466022.6
(22) Date of filing: 07.12.2012
(51) Int. Cl.: C05F 17/00, C05F 17/02, B65D 21/02, A01K 67/033

(54) **Dual-module vermicomposter**

(30) Priority: 15.12.2011 CZ 20110833
(71) Applicant: Ceská Zemedelská Univerzita V Praze, 165 21 Praha 6 Suchdol (CZ)
(72) Inventor: Pliva, Petr, 169 00 Praha 6 (CZ); Cejka, Zdenek, 161 00 Praha 6 (CZ); Hanc, Ales, 165 00 Praha 6 (CZ)
(74) Representative: Jirkalova, Hana

(57) **Abstract**

Dual-module vermireactor featuring two identical modules *"Module 1"* (2) and "*Module 2"* (3) arranged in two functional settings, either tightly connected or disconnected.

Each module is equipped with four rolling wheels (19), at the bottom of each module (2) and (3). Part of the bottom set is provided with a perforated metal sheet (9), with an underneath pull-out reservoir (10) fitted with a release valve (11). Each module is equipped with a main lid (12) and two side lids (13), a module panel (14) with technological opening (15). A main panel (16) is provided with a control unit and other elements for monitoring and management and is connected through data cables (17) and hoses (18) with the individual modules directly. The modules (2) and (3) are furhter equipped with a removable full wall (4), a perforated wall (5), fast-release clasps (6), alignment spikes (7) and rolling suspension (8).

## Description

### Technology Area

The solution deals with a dual-module vermireactor, describing a device in which bio-waste is processed in a closed environment with the use of epigeic earthworms, most often species *Eisenia fetida, Eisenia Andrei* or *Dendrobena Veneta* and a process known as vermicomposting.

### The Current State of Technology

The vermicomposting technology is currently used mostly in treatment of materials and waste coming from animal and plant agricultural production. In a smaller scale, it's also used in the treatment of kitchen waste in household vermicomposters. However, there are many more opportunities for use.

Recently, the opportunities to use vermicomposting in the treatment of communal waste (including the problematic gastronomic waste) are being verified. Also, the option of vermicomposting is being tested for the current method of anaerobic digestion for agricultural and non-agricultural waste, which produces a problematic digestate.

Successful vermicomposting requires ensuring optimal conditions. This particularly relates to the sufficient supply of materials and nutrients, moisture content within the environment (specifically of the treated materials), levels of aeration, temperatures in the earthworm environment, pH levels, salts content and other less important parameters. The design of various vermicomposting systems is closely related to these requirements.

As with traditional composting, there are also several types of technological systems of vermicomposting that differ in their technological procedures, each method offering several further process versions. Vermicomposting is usually a mass-production process, however, although there are also a number of small-scale vermicomposting operations. An example is treatment of kitchen waste in a household vermicomposter.

There is also vermicomposting in vermireactors, along with those in open-space and containers. Among the most significant benefits of this solution are the notable speed-up of the entire process, limiting the surface necessary for composting and the influence of weather conditions on the process, better use of the resulting leach and the opportunity to manage and automate the entire operation.

Currently, a whole range of various vermicomposters have the common ability to manage the vermicomposting process through feedback, under optimal conditions and based on monitoring certain physical values.

### The Essence of the Invention

The shortcomings touched on above are remedied by the invention of the dual-model vermireactor which is, in essence, composed of two equal modules - "Module 1" and "Module 2" - arranged in two work spaces and either tightly attached or disconnected.

The dual-module vermireactor is characterized by each module equipped with four rolling wheels. A part of the bottom of each module features perforated metal with a pull-out reservoir underneath, equipped with a release valve. Each module has a main lid and two additional lids, as well as the module panel with a technological opening, housing the main panel, control unit and other features for monitoring and management. The main panel is connected by data cables and hoses directly to the individual modules, specifically through the module panel. Both modules are equipped with a removable full wall, a perforated wall, fast-release clips, alignment spikes and rolling suspensions.

Unlike other vermireactors, the dual-module vermireactor is designed with two absolutely identical modules. The modules can function in two work settings - in the **disconnected setting,** where each module is used individually (one is being filled, while the vermicomposting process takes place in the other), or in the tight **connection setting.** Both modules are connected in case it's necessary to transfer earthworms from one module (in which the material is already processed to complete vermicompost) to the other module (where the materials are pre-composted). The vermireactor modules are connected for the time necessary to allow the earthworms to instinctively move through the aligned perforated walls between the individual modules and reach the food necessary for their survival.

The dual-module vermireactor is composed of two modules that can easily be connected. The individual modules (always equipped with one removable full and one removable perforated wall) easily connect and enable the free movement of earthworms between them. The modules are interchangeable, mobile and each module has a lid for supplying processed materials. After removal of the full and perforated walls, the finished vermicompost can be emptied from the module and it can be cleaned. A removable reservoir, with a vent for collecting the liquids released during the composting process is placed in the bottom part.

When the earthworms leave the module with finished vermicompost, both models are disconnected. In the module where earthworms remain, once the side wall is closed, the decomposing process of the materials begins. Meanwhile, the finished vermicompost is removed from the other and the module interior is cleaned, including the reservoir for the liquid part of vermicomposting. After covering the side wall, the module is again ready for the collection of biodegradable materials for treatment and the entire process repeats.

Earthworms must be provided with an optimal environment for vermicomposting. The assurance of optimal conditions within the compost environment particularly regards a sufficient supply of materials (and thus the sufficient supply of nutrients); moisture within the environment (specifically of the treated materials), aeration rates, temperature conditions where earthworms are present and other less important parameters. For these reasons, it is necessary to monitor the course of the composting process in the vermireactor by feedback-based management.

Each of the modules of the dual-module vermireactor has a module panel and technological opening enabling measurement and providing a sufficient amount of air, as well as regulation of both temperature and moisture content in the treated materials.

The dual-model vermireactor - whether with connected or disconnected modules - is equipped with a main panel with a control unit and other devices serving both modules.

The individual steps in composting in a dual-module vermireactor are as follows:
*(the initial position - both modules are set for individual function in a disconnected state - they are covered with full walls)*
   - **1)** to-be-treated materials are gradually added to *"Module 1",* if necessary, they are mixed and *"pre-composting"* takes place;
   - **2)** substrate with earthworms is added to *"Module 1"* (only inserted in this initial step - the earthworm substrate is not added in further cycles);
   - **3)** the earthworms compost the material *in "Module 1"*;
   - **4)** to-be-treated materials are gradually added to *"Module 2",* if necessary, they are mixed and *"pre-composting"* takes place;
   - **5)** after the vermicompost is created by the earthworms over a period of time (depending on the type of treated materials) in *"Module 1"* and after "pre-composting" takes place in *"Module 2,"* the full walls are detached from both modules and the modules are connected;
   - **6)** gradually, the earthworms start migrating for new food from *"Module 1"* to *"Module 2",*
   - **7)** after the complete migration of earthworms, the modules are disconnected and the full wall is mounted on *"Module* 2" while the perforated wall is detached from *"Module 1"*;
   - **8)** the earthworms compost the materials in *"Module 2"*;
   - **9)** the finished vermicompost is removed from *"Module 1,"* the interior of the module is cleaned, both perforated and full walls are attached;
   - **10)** to-be-treated materials are gradually added to *"Module 1",* if necessary, they are mixed and *"pre-composting"* takes place;
   - **11)** The entire process repeats.

The benefit of the dual-module vermireactor, according to the invention, is that the migration of earthworms is gradual from one module (where the material is already transformed to a finished vermicompost) into the second module (where the materials are pre-composted) without the necessity for search and capture of the earthworms. Additionally, it is an opportunity to compost problematic biodegradable waste in an enclosed space. The pre-composting and vermicomposting is conveniently monitored and managed through the same monitoring equipment.

### Overview of the Plans

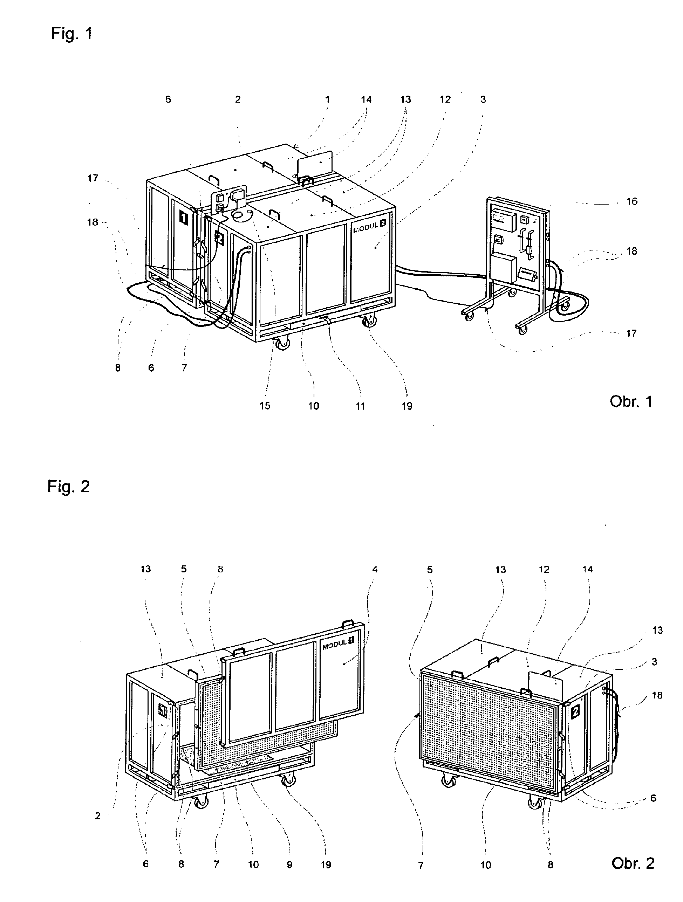

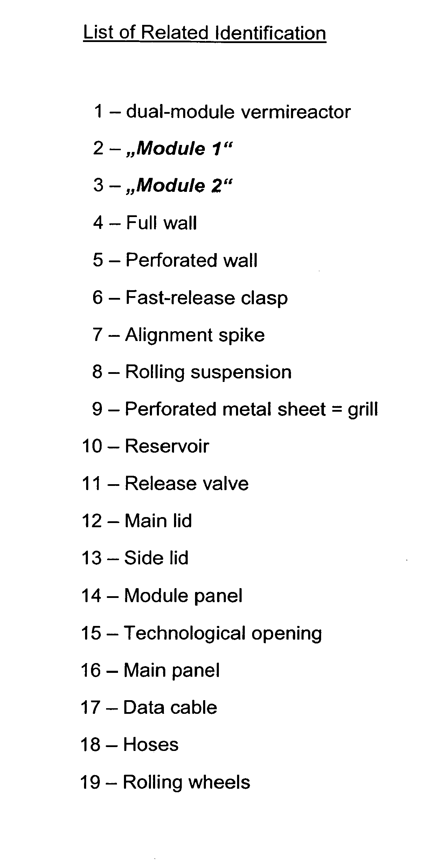

The attached plan schematically illustrates the operation of the dual-module vermicomposter according to the inventions. Figure 1 shows the dual-module vermicomposter in its connected state - "Module 1" and "Module 2" are tightly attached, including the main panel. Figure 2 shows the dual-module composted in its disconnected state.

### Operation Examples

### Example 1

The dual-model vermireactor 1 is composed to two modules - *"Module 1"* (2) and *"Module 2"* (3). Both modules, 2 and 3, are absolutely identical and can be arranged in two functional settings - either tightly connected (fig. 1) or disconnected (fig. 2).

In the tight attachment setting of both modules, it is necessary to move the earthworms. The connection of both modules is performed by removing the full wall on both modules 4, setting both modules with the perforated walls 5 next to each other and securing them with clasps 6. The connection of both modules is created in such ways that both perforated walls 5 are set next to each other so that the individual perforations align, which are secured by alignment spikes 7 and rolling suspensions 8.

After the module with the finished vermicompost is abandoned by the earthworms, both modules 2 and 3 are disconnected; the module with the earthworms (active) is covered by the full wall 4. The perforated wall 5 is removed from the other module, the finished vermicompost is collected and the interior of the module (including the reservoir 10 for the liquid component of vermicomposting) is cleaned. The walls are reattached, first the perforated wall 5 and then the full wall 4. The module is again ready for collection of to-be-treated biologically degradable materials and the whole process repeats.

In their disconnected setting, each module is used independently - one being filled while the vermicomposting process takes place in the other. Both modules are absolutely identical and interchangeable.

For reasons of mobility, particularly when connecting the individual modules 2 and 3, it is necessary that each module is equipped with four rolling wheels 19. At the bottom of each module, a part of the bottom is set with a perforated metal sheet - a grill 9 to enable drainage of the liquid component created during the start and course of composting. A pull-out reservoir 10 for the liquid vermicomposting component is placed beneath the module bottom - grill 9, which can be collected at certain times by use of release valve 11.

Each module 2 and 3 is equipped with a main lid 12 for supplying to-be-treated materials and two side lids 13 serving technological purposes (cleaning, mixing, interior control, insertion of sensors, and wiring connection).

To ensure optimal conditions for the vermicomposting process inside individual modules 2 and 3, the dual-module vermireactor 1 is equipped with a monitoring system and elements ensuring feedback-based management.

For these reasons, each of the modules 2 and 3 is set with a module panel 14 and a technological opening 15, through which it is possible to perform measurements in each module to sufficient amounts of air, temperature regulation and moisture content in the treated materials.

The dual-module vermireactor 1, according to the invention - whether with connected or disconnected modules 2 and 3 - is equipped with a main panel 16, on which the control unit and other elements for monitoring and management serving for both modules 2 and 3 is placed. The main panel 16 is connected through data cables 17 and hoses 18 directly with the individual modules 2 and 3, specifically through the module panel 14.

The dual-module vermireactor 1, according to the invention was successfully tested at the experimental work station of the FILIP company in Lu 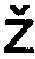 ice u Hodonina under professional supervision of the Czech University of Life Sciences Prague and the Research Institute of Agricultural Engineering, Prague, Czech Republic.

### Industrial Usability

The dual-module vermicomposter will find its use particularly with producers of biologically degradable waste that is difficult to process through open-space composting (gastronomic waste) - restaurants, cafeterias, small animal breeders, food processing operations, etc.

Its use is also possible in operations processing common (non-problematic) biologically degradable waste.

## Claims

1. The dual-module vermireactor, **featuring** two identical modules *"Module 1"* (2) and *"Module 2"* (3), arranged in two functional settings, either tightly connected or disconnected. Dual-module vermireactor according to the claim (1), is **characterized by each module** (2) and (3) being equipped with four rolling wheels (19), at the bottom of each module (2) and (3) there is a part of the bottom set with perforated metal sheet (9), under which there is pull-out reservoir (10) fitted with a release valve (11), while each module (2) and (3) is equipped with the main lid (12) and two side lids (13) as well as the module panel (14) with technological opening (15), the main panel (16) with control unit and other elements for monitoring and management, while the main panel (16) is connected through data cables (17) and hoses (18) with the individual modules directly, specifically through the module panel (14), while the modules (2) and (3) are equipped with a removable full wall (4), perforated wall (5), fast-release clasps (6), alignment spikes (7) and rolling suspension (8).
